# EUROPÄISCHE PATENTANMELDUNG

(11) **EP 3 884 840 A1**
(43) Veröffentlichungstag der Anmeldung: **29.09.2021**
(21) Anmeldenummer: 20166380.4
(22) Anmeldetag: 27.03.2020
(51) Int. Cl.: A61B 1/00, A61B 5/00, G01N 21/64, A61B 5/1455, A61B 5/1459, A61B 5/024

(54) **MEDIZINISCHE BILDGEBUNGSVORRICHTUNG ZUR ORTSAUFGELÖSTEN AUFNAHME MULTISPEKTRALER VIDEODATEN**

(71) Anmelder: Diaspective Vision GmbH, 18233 Pepelow (DE); Karl Storz SE & Co. KG, 78532 Tuttlingen (DE)
(72) Erfinder: Kulcke, Axel, 18233 Am Salzhaff/ OTPepelow (DE); Köhler, Hannes, 04299 Leipzig (DE); Holmer, Amadeus, 18209 Bad Doberan (DE)
(74) Vertreter: Uexküll & Stolberg

(57) **Zusammenfassung**

Medizinische Bildgebungsvorrichtung zur ortsaufgelösten Aufnahme multispektraler Videodaten eines Untersuchungsgebiets eines Patienten, mit
einer Lichtquelle (3) mit mehreren optischen Emittern mit unterschiedlichen Wellenlängen im sichtbaren und NIR-Spektralbereich,
einem RGB-Kamerasensor (4), der mit Filtern mit Rot-, Grün- und Blau-Filterkurven rote, grüne und blaue Farbsignale des Untersuchungsgebiets erzeugt,
einer Aufnahmesteuerungseinrichtung (5) zur synchronisierten Steuerung von Lichtquelle (3) und RGB-Kamerasensor (4), um einen oder mehrere Emitter, die zusammen ein spektrales Belichtungsmuster mit einer oder mehreren Wellenlängen erzeugen, in einer vorgegebenen Aktivierungssequenz mehrerer aufeinanderfolgender spektraler Belichtungsmuster, die jeweils als ein Bild durch den RGB-Kamerasensor aufgenommen werden, wiederholt zu aktivieren, und
einer mit der Aufnahmesteuerungseinrichtung (5) und dem RGB-Kamerasensor (4) verbundenen Datenverarbeitungseinrichtung (6, 7), die zur Aufnahme der Farbsignale des RGB-Kamerasensors und zu deren Auswertung zur ortsaufgelösten multispektralen Analyse zur Ableitung eines physiologischen Parameters und zu dessen ortsaufgelöster Darstellung als Video eingerichtet ist, dadurch gekennzeichnet, dass
die Lichtquelle (3) einen Emitter aufweist, dessen Wellenlänge im Bereich von ±50% seiner Halbwertsbreite um den Schnittpunkt der Blau- und Grün-Filterkurve oder der Grün- und Rot-Filterkurve liegt, und
die Aufnahmesteuerungs- (5) und die Datenverarbeitungseinrichtung (6, 7) dazu eingerichtet sind, in einem Belichtungsmuster mit Aktivierung des Emitters am Schnittpunkt die betroffen beiden der roten und grünen oder der grünen und blauen Farbsignale separat zu erfassen und diese in der multispektralen Analyse mit durch die beiden betroffenen Filterkurven gegeneinander verschobenen, voneinander verschiedenen Wellenlängen als zwei Stützstellen-Wellenlängen auszuwerten.

## Beschreibung

Die vorliegende Erfindung betrifft eine medizinische Bildgebungsvorrichtung zur ortsaufgelösten Aufnahme multispektraler Videodaten eines Untersuchungsgebiets eines Patienten, mit
einer Lichtquelle zur Beleuchtung des Untersuchungsgebiets, die mehrere optische Emitter mit unterschiedlichen Wellenlängen verteilt über den sichtbaren und NIR-Spektralbereich aufweist,
einem RGB-Kamerasensor zur Aufnahme des Untersuchungsgebiets, der mit Filtern mit Rot-, Grün- und Blau-Filterkurven rote, grüne und blaue Farbsignale erzeugt,
einer Aufnahmesteuerungseinrichtung, die zur synchronisierten Steuerung von Lichtquelle und RGB-Kamerasensor eingerichtet ist, um einen oder mehrere Emitter, die zusammen ein spektrales Belichtungsmuster mit einer oder mehreren Wellenlängen erzeugen, in einer vorgegebenen Aktivierungssequenz mehrerer aufeinanderfolgender spektraler Belichtungsmuster, die jeweils als Bild durch den RGB-Kamerasensor aufgenommen werden, wiederholt zu aktivieren,
einer mit der Aufnahmesteuerungseinrichtung und dem RGB-Kamerasensor verbundenen Datenverarbeitungseinrichtung zur Aufnahme der RGB-Kamerasensorsignale und zu deren Auswertung zur ortsaufgelösten multispektralen Analyse zur Ableitung eines physiologischen Parameters und zu dessen ortsaufgelöster Darstellung als Video des Untersuchungsgebiets.

Die Erfindung betrifft eine medizinische Bildgebungsvorrichtung, die in ein Endoskop, Laparoskop, Mikroskop oder Exoskop integriert sein kann. Die Aufnahme einer Videosequenz von Bildern erfolgt über einen oder zwei (Stereoskop) hochauflösende Farbkamerasensoren (RGB-Kamerasensoren) mit Farbfiltern mit Rot-, Grün- und Blau-Filterkurven auf Grundlage von CMOS- oder CCD-Technologie für den eigentlichen Sensor.

In der Medizin werden verschiedene optische Geräte eingesetzt, die Ärzte bei Diagnose und Therapie unterstützen. Diese Geräte verwenden häufig hochauflösende Farbkameras zur Aufnahme des Untersuchungsgebiets. Ein Beispiel ist das medizinische Videoendoskop. Am proximalen oder distalen Ausgang des Endoskops befindet sich ein Farbkamerasensor. Bei den Endoskopen setzt sich hier die "Chip in the tip" (CIT) Technologie immer weiter durch, bei der der miniaturisierte Farbkamerasensor mit einer abbildenden Optik direkt in der Spitze des Endoskops (distal) platziert ist. Die hier beschriebene Vorrichtung und des entsprechende Verfahren kann mit dieser Technologie genutzt werden. Die RGB-Farbkamerasensoren sind Farbfiltersensoren mit Rot-, Grün-, Blau-Farbfiltern, die in einer Bayer-Matrix angeordnet sind. Die Pixel hinter den Rot-, Grün-, Blau-Farbfiltern erzeugen rote, grüne und blaue Farbsignale. Die Beleuchtung erfolgt durch eine Lichtquelle (z.B. Kaltlichtquellen mit Halogenlampen oder Xenonlichtquellen, Laser oder vorzugsweise LED-Lichtquellen), wobei das Licht der Lichtquelle üblicherweise über Glasfaseroptiken in das Endoskop oder ein anderweitiges optisches Geräte eingekoppelt wird und das Untersuchungsgebiet beleuchtet. Solche Endoskope können mit sehr hochauflösender (Ultra-HD) und/oder dreidimensionaler Bildgebung (Stereoskope) ausgestaltet sein.

In WO 2017/118735 A2 ist ein am menschlichen Körper anzuwendendes, multispektrales Analyseverfahren beschrieben, bei dem eine Lichtquelle mit mehreren LEDs unterschiedlicher Wellenlängen im sichtbaren und im NIR-Spektralbereich und ein Kamerasensor verwendet werden; das Verfahren ist allerdings kein bildgebendes Verfahren, sondern dient zur Ermittlung einzelner physiologischer Parameter eines Probanden durch multispektrale Analyse. Die Lichtquelle wird wiederholt in Aktivierungssequenzen mit aufeinanderfolgenden spektralen Belichtungsmustern bei Aktivierung von LEDs mit unterschiedlichen Wellenlängen belichtet. Die aus der multispektralen Analyse gewonnenen Parameter werden aber nicht ortsaufgelöst dargestellt. Vielmehr ist der Kamerasensor direkt auf die Haut des Probanden aufgesetzt und erfasst durch das Gewebe des Probanden transflektiertes Licht als Funktion des Abstands von der Lichtquelle zum Erfassungsort, um daraus physiologische Parameter des Probanden bei körperlicher Belastung abzuleiten, wozu Sauerstoffsättigung des arteriellen Blutes, Gewebesauerstoffsättigung, Wassergehalt, Pulsfrequenz, Wassergehalt des Gewebes etc. gehören.

Außerhalb der medizinischen Bildanalyse gibt es eine Reihe von Bildanalyseverfahren, die LEDs verschiedener Wellenlängen mit Belichtung mit verschiedenen Wellenlängen im synchronisierten Multiplexbetrieb mit einem Farbsensor zur Ermittlung spektraler Informationen anwenden, siehe z.B. US 2016/0187199 A1, US 10 161 796 B1 und US 2010/0073504 A1.

Klassische Videoendoskope dienen zur Farbvideodarstellung eines Untersuchungsgebietes im Körperinneren. Aus der physiologischen Bildgebung ist nun bekannt, dass den Nutzern der Endoskope eine spektrale Bildgebung, hier multi- oder hyperspektrale Bildgebung, viele zusätzliche Informationen liefern kann, die bei Operationen oder in der Diagnostik nutzbar gemacht werden können. Ein sehr wichtiger Bereich in der physiologischen Bildgebung ist die Perfusionsbildgebung, die zum Beispiel über die multispektrale Analyse der Farbsignale oder durch die Fluoreszenzbildgebung durchgeführt werden kann.

In der Medizintechnik ist die physiologische Bildgebung mit multispektralen oder hyperspektralen Methoden unter anderem dafür bekannt, dass dadurch ortsaufgelöst durch Falschfarben dargestellte physiologische Parameter, wie Hämoglobingehalt und die Oxygenierung des Hämoglobins im Untersuchungsgebiet, für den Mediziner sehr wichtige klinische Informationen enthalten. Weiterhin ist die ortsaufgelöste spektrale Bildgebung auch als molekulare Bildgebung unter Verwendung chemometrischer Verfahren bekannt, da chemische Informationen ortsaufgelöst darstellbar sind. Für die klinische Anwendung sind die ortsaufgelöste Darstellung des Hämoglobingehalts besonders der Wassergehalt und der Fettanteil im Gewebe wichtige Größen.

Auch die Ableitung systemischer physiologischer Parameter wie der Herzfrequenz, der Atemfrequenz und vor allem der Eigenschaften des arteriellen Blutes ist von großer Bedeutung und liefert Informationen mit hoher Aussagekraft über den Zustand des Patienten. Es gibt bereits verschiedene Ansätze um die ortsaufgelöste Bildgebung eines physiologischen Parameters mit der üblichen Weißlichtbildgebung (Farbvideo) zu kombinieren.

Außerhalb des medizinischen Bereiches gibt es eine Reihe von Bildgebungsverfahren unter Anwendung multispektraler Analyse, wobei zur Gewinnung von ortsaufgelösten, multispektralen Informationen LED-Lichtquellen ein Objekt oder einen Untersuchungsgegenstand mit LED-Emittern verschiedener Wellenlängen im Multiplexbetrieb wiederholt mit Aktivierungssequenzen mit aufeinanderfolgenden verschiedenen spektralen Belichtungsmustern beleuchten und mit einem Farbkamerasensor reflektiertes Licht aufnehmen, wobei die Lichtquelle so getaktet und synchronisiert mit dem Betrieb des Farbkamerasensors ist, dass aufeinanderfolgende Belichtungsmuster in aufeinanderfolgend aufgenommene Bilder (Video-Frames) des Farbkamerasensors fallen. Die verschiedenen spektralen Belichtungsmuster einer Aktivierungssequenz können so separat erfasst und einer multispektralen Analyse unterzogen werden.

Aus WO 2019/051591 A1 ist eine medizinische Bildgebungsvorrichtung mit den Merkmalen des Oberbegriffs von Patentanspruch 1 bekannt. Die verwendete Lichtquelle umfasst eine Mehrzahl von LEDs mit verschieden Wellenlängen im sichtbaren und im NIR-Spektralbereich. Da eine Aktivierungssequenz aufeinanderfolgender spektraler Belichtungsmuster mit einer entsprechenden Anzahl von aufeinanderfolgenden Bildern mit dem Farbkamerasensor aufgenommen wird, ist nach der multispektralen Analyse die Bildrate, mit der die aus der multispektralen Analyse gewonnene physiologische Information dargestellt wird, gegenüber der Bildrate des Farbkamerasensors für ein normales Farbvideo entsprechend der Anzahl der aufeinanderfolgenden Belichtungsmuster in der Aktivierungssequenz reduziert. Um trotzdem eine hinreichende Zeitauflösung der als Video in Falschfarben dargestellten physiologischen Information aus der multispektrale Analyse zu ermöglichen, werden in dem beschriebenen Verfahren Belichtungsmuster verwendet, in denen mehrere LEDs bei verschiedenen Wellenlängen gleichzeitig aktiviert werden. Die den unterschiedlichen Wellenlängen zugeordneten Intensitätsinformationen können aus den drei Farbsignalen des Farbkamerasensors voneinander separiert werden, wobei solche Techniken als spektrale Entmischung (spectral unmixing) bezeichnet werden. Damit ist es möglich, pro Bild, das von dem Farbkamerasensor aufgenommen wird, spektrale Informationen bei zwei oder mehr Wellenlängen zu erfassen. Auf diese Weise können Aktivierungssequenzen mit z.B. zwei oder drei aufeinanderfolgenden spektralen Belichtungsmustern mit jeweils simultaner Aktivierung mit mehreren Wellenlängen ausreichend sein, um mit einer hinreichenden Anzahl spektraler Stützstellen bei verschiedenen Wellenlängen aussagekräftige multispektrale Analysen durchzuführen.

Wenn Aktivierungssequenzen z.B. mit drei aufeinanderfolgenden spektralen Belichtungsmustern aufeinanderfolgend wiederholt werden, und nach der darauf basierenden multispektralen Analyse jeweils ein Bild des abgeleiteten physiologischen Parameters in Falschfarben dargestellt, hat das Video des physiologischen Parameters, z.B. der Sauerstoffsättigung des arteriellen Blutes, eine Bildrate, die gegenüber einem normalen Farbvideo auf 1/3 reduziert ist.

Für ein hochwertiges Farbvideo ist heute eine Bildwiederholrate von 60 Hz Standard. Entsprechend sind die heute weit verbreiteten RGB-Kamerasensoren für eine solche Bildwiderholrate von 60 Hz ausgelegt, wobei einige Kamerasensoren auch Wiederholraten bis zu 120 Hz erreichen können. Für die physiologische Bildgebung aus multispektraler Analyse abgeleiteter physiologischer Parameter ist eine auf 1/3 reduzierte Bildwiederholrate, also typischerweise 20 Hz, akzeptabel.

Es ist Aufgabe der vorliegenden Erfindung, eine Vorrichtung und ein Verfahren zur medizinischen Bildgebung eines aus einer multispektralen Analyse abgeleiteten physiologischen Parameters anzugeben, wobei bei gegebener Anzahl von spektralen Belichtungsmustern pro Aktivierungssequenz eine möglichst große Anzahl von spektralen Stützstellen (Anzahl von unterschiedlichen Wellenlängen) für die multispektrale Analyse bereitgestellt werden sollen.

Zur Lösung dieser Aufgabe dienen die medizinische Bildgebungsvorrichtung mit den Merkmalen des Patentanspruchs 1 und das Verfahren mit den Merkmalen des Patentanspruchs 14. Vorteilhafte Ausführungsformen sind in den Unteransprüchen angegeben.

Die medizinische Bildgebungsvorrichtung hat eine Lichtquelle mit einem Emitter, typischerweise eine LED, mit einer Wellenlänge, die im Bereich von ±50% der Halbwertsbreite des Emitters um den Schnittpunkt der Blau- und Grün-Filterkurve oder der Grün- und Rot-Filterkurve liegt. Als Wellenlänge wird hier die Spitzen- oder Peak-Wellenlänge des Emissionsspektrums des Emitters bezeichnet. Der zuerst genannte Schnittpunkt ist der Schnittpunkt des nach dem Maximum abfallenden Verlaufs der Blau-Filterkurve mit dem vor ihrem Maximum ansteigenden Verlauf der Grün-Filterkurve und der zweite genannte Schnittpunkt ist der Schnittpunkt des nach ihrem Maximum abfallenden Verlaufs der Grün-Filterkurve mit dem vor ihrem Maximum ansteigenden Verlauf der Rot-Filterkurve, d.h. es sind die Schnittpunkte im jeweiligen Übergangsbereich der genannten Filterkurven gemeint. Mit anderen Worten ist die Spitzenwellenlänge des Emitters um nicht mehr als die Hälfte der Halbwertsbreite des Emitters gegenüber der Wellenlänge des Schnittpunkts der betroffenen Filterkurven verschoben. Anders ausgedrückt liegt die Wellenlänge des Schnittpunkts der betroffenen beiden Filterkurven in dem von der Halbwertsbreite des Emitters überstrichenen Wellenlängenintervall. Dadurch, dass die Wellenlänge des Emitters ausreichend nahe, nämlich nicht weiter als 50% der Halbwertsbreite vom Schnittpunkt der betroffenen beiden Filterkurven entfernt ist, ist sichergestellt, dass auf beide Farbsignale, die zu den betroffenen Filterkurven gehören, ein signifikanter Anteil der abgestrahlten Intensität des Emitters entfällt.

Die Aufnahmesteuerungs- und die Datenverarbeitungseinrichtung sind dazu eingerichtet, in einem spektralem Belichtungsmuster mit Aktivierung eines Emitters an einem der Schnittpunkte die betroffenen Beiden der roten und grünen oder der grünen und blauen Farbsignale separat zu erfassen und diese in der multispektralen Analyse mit durch die beiden betroffenen Filterkurven gegeneinander verschobenen, voneinander verschiedenen Wellenlängen zwei Stützstellen-Wellenlängen auszuwerten. Auf diese Weise können durch die beiden betroffenen Filterkurven bewirkte Verschiebung der Signale eines Emitters in den beiden betroffenen Farbsignalen durch einen Emitter zwei Farbsignale mit gegeneinander verschobenen Spitzenwellenlängen aufgenommen werden. Dadurch ist es möglich, durch Aktivierung eines einzelnen Emitters zwei Stützstellen-Wellenlängen für die multispektrale Analyse zu erhalten.

In einer bevorzugten Ausführungsform weist die Lichtquelle einen ersten Emitter, dessen Wellenlänge im Bereich von ±50% seiner Halbwertsbreite um den Schnittpunkt der Blau- und Grün-Filterkurve liegt, und einen zweiten Emitter auf, dessen Wellenlänge im Bereich von ±50% seiner Halbwertsbreite um den Schnittpunkt der Grün- und Rot-Filterkurve liegt. Die Aufnahmesteuerungs- und die Datenverarbeitungseinrichtung sind dazu eingerichtet, den ersten und den zweiten Emitter in verschiedenen Belichtungsmustern der Aktivierungssequenz zu aktivieren und bei Aktivierung des ersten Emitters die gründen und roten Farbsignale separat zu erfassen und diese in der multispektralen Analyse mit durch die Blau- und Grün-Filterkurve gegeneinander verschoben, voneinander verschiedenen Wellenlängen als zwei Stützstellen-Wellenlängen auszuwerten und bei Aktivierung des zweiten Emitters die grünen und roten Farbsignale separat zu erfassen und diese in der multispektralen Analyse mit durch die Grün- und Rot-Filterkurve gegeneinander verschobenen, voneinander verschiedenen Wellenlängen als zwei Stützstellen-Wellenlängen auszuwerten. Auf diese Weise werden durch den ersten und den zweiten Emitter insgesamt vier verschiedene Wellenlängen für die multispektrale Analyse erfasst.

In einer weiteren Ausführungsform weist die Lichtquelle einen ersten Emitter auf, dessen Wellenlänge um weniger als ±50% seiner Halbwertsbreite kleiner als die Wellenlänge am Schnittpunkt der Blau- und Grünfilterkurve ist, und einen zweiten Emitter auf, dessen Wellenlänge um weniger als ±50% seiner Halbwertsbreite größer als die Wellenlänge am Schnittpunkt der Blau- und Grün-Filterkurve ist. Die Aufnahmesteuerungs- und Datenverarbeitungseinrichtung sind dazu eingerichtet, den ersten und den zweiten Emitter in verschiedenen Belichtungsmustern der Aktivierungssequenz zu aktivieren und sowohl bei Aktivierung des erste Emitters als auch bei Aktivierung des zweiten Emitters jeweils die grünen und blauen Farbsignale separat zu erfassen und diese in der multispektralen Analyse jeweils mit durch die Blau- und Grün-Filterkurve gegeneinander verschoben sind, voneinander verschiedenen Wellenlängen als insgesamt vier Stützstellen-Wellenlängen auszuwerten.

Auch auf diese Weise können für die multispektrale Analyse vier Stützstellen-Wellenlängen im Blau-Grün-Übergangsbereich erzeugt werden.

In einer bevorzugten Ausführungsform sind die Aufnahmesteuerungs- und die Datenverarbeitungseinrichtung dazu eingerichtet, bei Steuerung der Lichtquelle mit einem Belichtungsmuster mit einem oder mehreren Emittern mit Wellenlängen im sichtbaren Bereich gleichzeitig einen Emitter im NIR-Spektralbereich zu aktivieren und dasjenige von den blauen, grünen und roten Farbsignalen, auf das die Belichtung mit den Emittern im sichtbaren Bereich den geringsten Einfluss hat, von den beiden anderen Farbsignalen zu subtrahieren, um den Effekt der Belichtung im NIR-Spektralbereich auf die beiden andere Farbsignale zu kompensieren. Die Rot-, Grün- und Blau-Filterkurven haben jeweils neben ihrer Hauptfilterkurve im roten, grünen bzw. blauen Spektralbereich im NIR-Spektralbereich einen in gleicher Weise wieder ansteigenden Filterkurvenverlauf. Dies hat zur Folge, dass bei einer Belichtung mit einer Wellenlänge im NIR-Spektralbereich alle drei Farbsignale näherungsweise in gleicher Stärke erhöhte Farbsignale haben. Dasjenige Farbsignal, das von der gleichzeitigen Belichtung im sichtbaren Bereich am wenigsten beeinflusst wird, idealerweise vernachlässigbar wenig beeinflusst wird, kann dann als Korrektur von den beiden anderen verbleibende Farbsignalen abgezogen werden, um den Beitrag der Belichtung im NIR-Bereich daran zu kompensieren.

Bei einer solchen Ausführungsform können die Aufnahmesteuerungs- und Datenverarbeitungseinrichtung dazu eingerichtet sein, bei Steuerung der Lichtquelle zur Erzeugung eines Belichtungsmusters mit Wellenlängen oberhalb von 500 nm gleichzeitig einen ersten Emitter mit einer Wellenlänge im Bereich von 500 bis 600 nm, einen zweiten Emitter mit einer Wellenlänge im Bereich von 600 bis 700 nm und einen Emitter mit Wellenlänge im NIR-Spektralbereich zu aktivieren und dann das blaue Farbsignal, das von den Belichtungen im sichtbaren Bereich am wenigstens beeinflusst ist, von den grünen und dem roten Farbsignal zu subtrahieren, um den Effekt der Belichtung im NIR-Spektralbereich auf das grüne und das rote Farbsignal zu kompensieren.

Im medizinischen Betrieb ist es wichtig, neben der bildlichen Darstellung eines aus der multispektralen Analyse abgeleiteten physiologischen Parameters das Untersuchungsgebiet auch in Form eines Farbvideos parallel zu dem Videobildern des physiologischen Parameters anzuzeigen. Aus diesem Grund ist in einer bevorzugten Ausführungsform die Aufnahmesteuerungseinrichtung dazu eingerichtet, zwischen einem Weißlicht-Betriebsmodus und einem multispektralen Betriebsmodus hin und her schaltbar zu sein. In dem Weißlicht-Betriebsmodus wird eine Mehrzahl von Emittern mit Wellenlängen verteilt über den sichtbaren Spektralbereich gleichzeitig kontinuierlich aktiviert, um eine kontinuierliche Beleuchtung mit einem näherungsweise Weißlicht entsprechenden Spektrum zu bewirken, und dabei die roten, grünen und blauen Farbsignale des RGB-Kamerasensors aufzunehmen und diese als Farbvideo zur Darstellung zu bringen. Im multispektralen Betriebsmodus ist die Aufnahmesteuerungseinrichtung dazu eingerichtet, in der Aktivierungssequenz Belichtungsmuster mit aktivierten Emittern zu selektieren, die bei Summation ein angenähertes Weißlichtspektrum ergeben, und die zugehörigen summierten roten, grünen und blauen Farbsignale als Farbvideo mit gegenüber dem Farbvideo im Weißlicht-Betriebsmodus reduzierter Bildrate parallel neben dem ortsaufgelösten Video des durch die multispektrale Analyse abgeleiteten physiologischen Parameters darzustellen. Auf diese Weise werden Belichtungsmuster mit Wellenlängen im sichtbaren Bereich sowohl zur Erzeugung eines Farbvideos als auch zur Erzeugung der Videodarstellung des aus der multispektralen Analyse abgeleiteten physiologischen Parameters verwendet.

In einer bevorzugten Ausführungsform sind die Aufzeichnungssteuerungs- und die Datenverarbeitungseinrichtung dazu eingerichtet, die im Zuge der multispektralen Analyse für mehrere Wellenlängen bestimmten Intensitäten *I_{λi}*(*t*) jeweils über die Belichtungsmuster mehrerer aufeinanderfolgender Aktivierungssequenzen zu einem zeitlichen Mittel *̅I̅_̅{̅λ̅i̅}̅*̅(̅*̅t̅*̅)̅ zusammenzufassen, daraus den Graphen eines Gewebespektrums mit den Stützstellen der mehreren Wellenlängen *λᵢ* zu bilden und daraus als physiologischen Parameter einen nicht-pulsatilen, gewebespezifischen Parameter abzuleiten. Als nicht-pulsatile Parameter können beispielsweise die Sauerstoffsättigung der Mikrozirkulation im Gewebe, der Gewebehämoglobingehalt, der Gewebewassergehalt oder der Gewebefettgehalt bestimmt werden.

In einer bevorzugten Ausführungsform sind die Aufnahmesteuerungs- und die Datenverarbeitungseinrichtung dazu eingerichtet, die im Zuge der multispektralen Analyse für mehrere Wellenlängen *λᵢ* bestimmten spektralen Intensitäten für eine erste Aktivierungssequenz als *I*_{*t*1}(*λᵢ*) zu erfassen und für eine spätere, zweite Aktivierungssequenz als *I*_{*t*2}(*λᵢ*) zu erfassen und den Graphen der an den Stützstellen-Wellenlängen *λᵢ* gebildeten Differenz der Intensitäten *I*_{*t*1}(*λᵢ*) und *I*_{*t*2}(*λᵢ*) als Funktion von *λᵢ* auszuwerten, um einen pulsatilen Parameter als physiologischen Parameter zu bestimmen. Der zeitliche Abstand der ersten und zweiten Aktivierungssequenzen kann so gewählt sein, dass sie innerhalb einer Periodendauer des Herzpulses liegen; z.B. kann eine Differenz zwischen einer Aktivierungssequenz nahe am Hochpunkt und einer Aktivierungssequenz nahe am Tiefpunkt des Pulssignals gebildet und ausgewertet werden. Zur Ermittlung von sich relativ zum Pulsschlag langsam ändernden physiologischen Parametern, wie etwa der Sauerstoffsättigung des arteriellen Blutes, können auch periodenübergreifende Differenzen von Intensitätspektren *Iₜᵢ*(*λᵢ*) aus verschieden Perioden des Pulsationssignals gebildet und ausgewertet werden. Es können auch zwei oder mehr Differenzen zwischen Intensitätspektren *Iₜᵢ*(*λᵢ*) in verschiedenen der aufeinanderfolgenden Aktivierungssequenzen aus einer oder mehreren Perioden des Pulsationssignals gebildet und ausgewertet werden.

Als pulsatile Parameter können so z.B. die Sauerstoffsättigung des arteriellen Blutes, die Herzfrequenz, der Pulsationsindex oder die Herzfrequenzvariabilität bestimmt werden.

In einer bevorzugten Ausführungsform sind zwei RGB-Kamerasensoren vorhanden und so zueinander angeordnet, um stereoskopische Aufnahmen zu ermöglichen.

Die Datenverarbeitungseinrichtung ist dazu eingerichtet, die Signale der beiden RGB-Kamerasensoren so auszuwerten, dass ein stereoskopisches Farbvideo zur Darstellung gebracht wird und eine stereoskopische Darstellung des durch die multispektrale Analyse ortsaufgelöst abgeleiteten physiologischen Parameters zur Anzeige gebracht wird.

In einer bevorzugten Ausführungsform weist die medizinische Bildgebungsvorrichtung eine Anregungslichtquelle zur Beleuchtung des Untersuchungsgebiets mit Anregungslicht im blauen oder im violetten Spektralbereich auf. Die Anregungslichtquelle ist so ausgestalt, dass ihr Spektrum des Anregungslichts von einer der Rot- und Grün-Filterkurven blockiert wird, so dass demgegenüber längerwelliges Fluoreszenzlicht von dem RGB-Kamerasensor ungestört durch das Anregungslicht erfassbar ist. In einer bevorzugten Ausführungsform weist die medizinische Bildgebungsvorrichtung ein Endoskop auf, das mit seinem distalen Ende die Lichtquelle und den RGB-Kamerasensor trägt, die über durch das Endoskop hindurch geführte und aus seinem proximalen Ende austretende Leitungen mit der Aufnahmesteuerungseinrichtung und der Datenverarbeitungseinrichtung in Datenaustauschverbindung stehen.

Schließlich wird durch die Erfindung ein Verfahren zur Aufnahme multispektraler Videodaten eines Untersuchungsgebiets eines Patienten, zur multispektralen Analyse zur ortsaufgelösten Ableitung eines physiologischen Parameters und zu dessen ortsaufgelöster Darstellung als Video des Untersuchungsgebiets bereitgestellt, wobei bei dem Verfahren das Untersuchungsgebiet mit einer Lichtquelle, die mehrere optische Emitter mit unterschiedlichen Wellenlängen verteilt über den sichtbaren und NIR-Spektralbereich aufweist, beleuchtet wird, das Untersuchungsgebiet mit einem RGB-Kamerasensor, der mit Filtern mit Rot-, Grün- und Blau-Filterkurven rote, grüne und blaue Farbsignale erzeugt, aufgenommen wird, die Lichtquelle und der RGB-Kamerasensor durch eine Aufnahmesteuerungseinrichtung synchronisiert gesteuert betrieben werden, um einen oder mehrere Emitter der Lichtquelle jeweils zur Erzeugung eines spektralen Belichtungsmusters mit einer oder mehreren Wellenlängen zu aktivieren, und in einer vorgegebenen Aktivierungssequenz mehrere aufeinanderfolgende vorgegebene Belichtungsmuster zu erzeugen, deren Reflexion im Untersuchungsgebiet jeweils als ein Bild pro Belichtungsmuster von dem RGB-Kamerasensor aufgenommen wird, und die Farbsignale des RGB-Kamerasensors in einer Datenverarbeitungseinrichtung, die mit der Aufnahmesteuerungseinrichtung und über diese mit dem RGB-Kamerasensor verbunden und dazu eingerichtet ist, einer ortsaufgelösten multispektralen Analyse unterzogen werden, um einen physiologischen Parameter abzuleiten, und Videodaten zur ortsaufgelöster Darstellung des Parameters als Video des Untersuchungsgebiets zu erzeugen, dadurch gekennzeichnet, dass ein Emitter der Lichtquelle, dessen Wellenlänge im Bereich von ±50% seiner Halbwertsbreite um die Wellenlänge des Schnittpunkts der Blau- und Grün-Filterkurve oder der Grün- und Rot-Filterkurve liegt, in einem Belichtungsmuster aktiviert wird und die betroffenen beiden Farbsignale, die den beiden Filterkurven des Schnittpunkts, in dessen Bereich der Emitter liegt, zugeordnet sind, in einem solchen Belichtungsmuster durch die Datenverarbeitungseinrichtung in der multispektraler in Analyse mit durch die beiden betroffenen Filterkurven gegeneinander verschobenen, voneinander verschiedenen Wellenlängen als zwei Stützstellen-Wellenlängen ausgewertet werden.

Durch Normierung der Intensitätssignale der verschiedenen Emitter auf eine Referenz (Weißabgleich) können aus den gemessenen Intensitäten die Datenpunkte des Reflexionsprofils und des Absorptionsprofils des betrachteten Untersuchungsgebiets an jedem Bildpunkt berechnet werden. Somit sind die Methoden aus der Spektroskopie und der multispektrale/hyperspektralen Kameratechnik für die Erzeugung der physiologischen Parameterbilder einsetzbar.

Ein weiterer Aspekt, der mit der medizinischen Bildgebungsvorrichtung nutzbar ist, ist die zeitaufgelöste Auswertung der medizinischen Signale. In diesem Zusammenhang ist besonders die Technologie der Pulsoxymetrie hervorzuheben, bei der eine pulsatile Auswertung der Signale die Oxygenierung des arteriellen Blutes erfasst werden kann. Derartige Auswertungen mit pulsatiler zeitlicher Auflösung können zur Visualisierung des Pulses und atmungsabhängiger physiologischer Parameter mit hoher räumlicher Auflösung verwendet werden.

Die Pulsation des Blutes durch den Herzschlag und die resultierende zeitliche Änderung der Hämoglobinkonzentration an einer lokalen Messstelle ist die Grundlage der Pulsoxymetrie. Die Pulsoxymetrie wertet konventionell die pulsatile Änderung der Absorption des Lichts im Gewebe bei 660 nm und 910 nm aus. Die Lichtquelle zur Beleuchtung ist vorzugsweise mit Emittern in Form von LEDs ausgestattet, die zeitlich schnell getaktet werden können. Durch die schnelle Taktung kann das pulsierende Signal des arteriellen Blutes innerhals des Gewebes in einen variablen und einen gleichbleibenden Anteil zerlegt werden. Die Analyse des variablen Anteils mit den beiden genannten Wellenlängen, an denen sich die Intensitäten des gesättigten und ungesättigten Hämoglobins gegenläufig verhalten, erlaubt die Berechnung der Sauerstoffsättigung des arteriellen Blutes (SpO₂).

Bei einer solchen Auswertung können auch neben den beiden genannten Wellenlängen weitere spektrale Stützstellen verwendet werden.

Die Erfindung wird im Folgenden anhand von Ausführungsbeispielen im Zusammenhang mit den Zeichnungen beschrieben, in denen:
Fig. 1 schematisch den Aufbau einer erfindungsgemäßen medizinischen Bildgebungsvorrichtung zeigt,
Fig. 2 schematisch den Aufbau einer stereoskopischen Ausführungsform der medizinischen Bildgebungsvorrichtung zeigt,

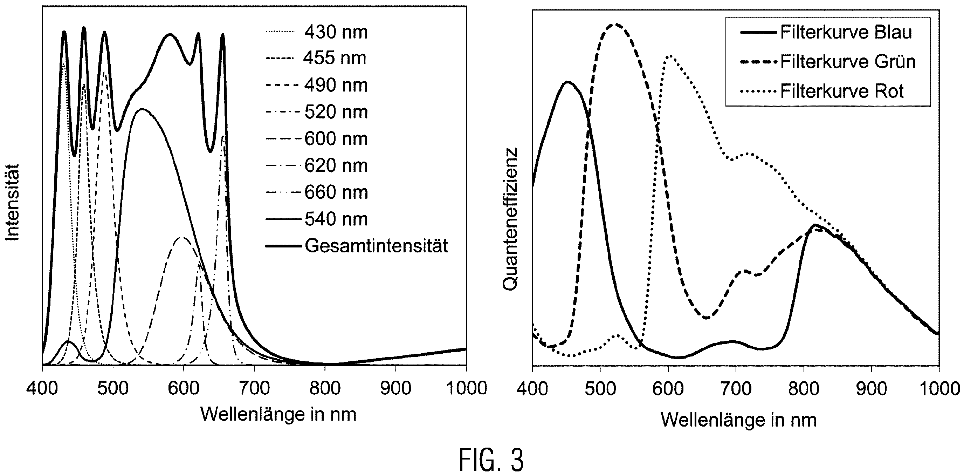

Fig. 3 in dem Graphen links die Emissionsspektren einer Anzahl von Emittern im sichtbaren Bereich und das aus diesem durch Überlagerung resultierende Gesamtspektrum zeigt und im Graphen rechts die Blau-, Grün- und Rot-Filterkurven eines RGB-Kamerasensors zeigt,
Fig. 4 die spektrale Emissionskurve eines breitbandigen orangen LED-Emitters, die drei Farbfilterkurven des RGB-Kamerasensors und die resultierende spektrale Signalverteilung im grünen und im roten Farbsignal zeigt,
Fig. 5 links eine Aktivierungssequenz mit zwei aufeinanderfolgenden spektralen Belichtungsmustern, in denen jeweils mehrere Emitter Lichtimpulse emittieren, und im rechten Teil die Emissionsspektren des ersten Belichtungsmusters (S 1 - oben) und des zweiten Belichtungsmusters (S 2 - unten) jeweils zusammen mit den Rot-, Grün- und Blau-Filterkurve des RGB-Kamerasensors zeigt,
Fig. 6 die zeitliche Abfolge von Belichtung und Auslesen des RGB-Kamerasensors mit Rolling-Shutter darstellt,
Fig. 7a - 7c jeweils links die Emissionsspektren eines spektralen Belichtungsmusters einer Aktivierungssequenz mit drei aufeinanderfolgenden spektralen Belichtungsmustern (Fig. 7a, 7b und 7c) jeweils zusammen mit den drei Farbfilterkurven zeigt, jeweils in der Mitte zwei resultierende Farbsignale und in der rechten Spalte jeweils das dritte resultierenden Farbsignal zeigt,
Fig. 8 oben die Emissionsspektren einer Reihe von LED-Emittern im sichtbaren Bereich zeigt und unten die resultierenden roten, grünen und blauen Farbsignale zeigt,
Fig. 9 einen Graphen der Aktivierungssequenz mit den drei aufeinanderfolgenden spektralen Belichtungsmustern S 1, S 2 und S 3 als Funktion der Zeit für die in Fig. 7 dargestellte Aktivierungssequenz zeigt,
Fig. 10 in der mittleren Spalte von oben nach unten die roten, grünen, blauen Farbsignale und darunter die roten und blauen Farbsignale für sieben aufeinanderfolgende Aktivierungssequenzen mit jeweils zwei aufeinanderfolgenden spektralen Belichtungsmustern gemäß Fig. 5 und daneben und darunter Zusammenfassungen der Signale zeigt,
Fig. 11 die Absorptionskurven der wichtigsten Absorber des Gewebes und die effektiven Stützstellen-Wellenlängen für die multispektrale Analyse darstellt und
Fig. 12 schematisch die Zusammensetzung der Gewebeabsorption und die daraus abgeleiteten physiologischen Parameter darstellt.

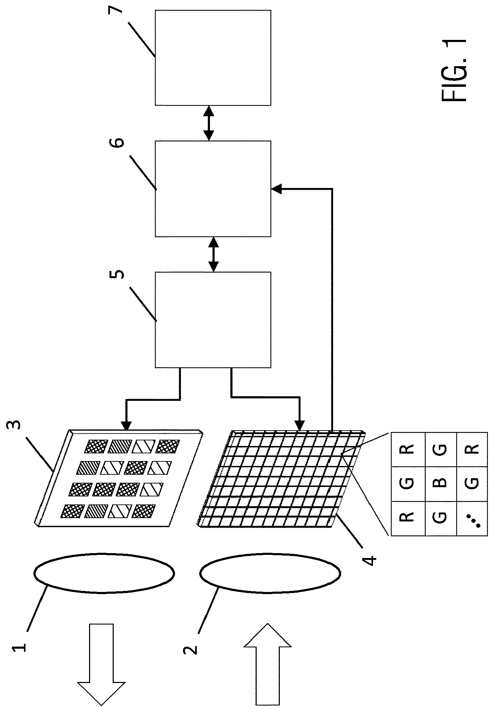

Fig. 1 zeigt als schematisches Blockschaltbild den Aufbau einer medizinischen Bildgebungsvorrichtung, die gemäß der vorliegenden Erfindung betreibbar ist. Die Bildgebungsvorrichtung umfasst eine Lichtquelle 3, die schematisch als Matrix mit mehreren in unterschiedlichen Grautönen dargestellter Emittern gezeigt ist. Die Emitter können durch LEDs gebildet sein, deren Wellenlängen über den sichtbaren und den NIR-Spektralbereich verteilt sind. Soweit hierin die Formulierung gebraucht wird, dass die Emitter jeweils "eine" Wellenlänge haben, ist damit die Spitzen- oder Peak-Wellenlänge des Emissionsspektrums des jeweiligen Emitters gemeint. Weitere Einzelheiten zu der Lichtquelle sind weiter unten beschrieben.

Das Licht der Lichtquelle 3 wird über eine Abbildungsoptik 1 auf das Untersuchungsgebiet gerichtet. Das vom Untersuchungsgebiet reflektierte Licht wird über ein Objektiv 2 auf einen RGB-Farbkamerasensor 4 gerichtet. Die mit R, G und B bezeichneten roten, grünen und blauen Farbfilter sind im sogenannten Bayer-Muster direkt vor den Pixeln des Kamerasensors angeordnet, der als CMOS- oder CCD-Sensor ausgeführt sein kann. Jedes Pixel misst daher direkt nur eines der drei Farbsignale, die beiden fehlenden Farben werden üblicherweise aus den Farbwerten der acht benachbarten Pixel geschätzt (interpoliert).

Eine Aufnahmesteuerungseinrichtung 5 kommuniziert mit der Lichtquelle 3 und dem RGB-Farbkamerasensor 4. Die Aufnahmesteuerungseinrichtung 5 ist dazu eingerichtet, den Betrieb von der Lichtquelle 3 und den Betrieb des Farbkamerasensors 4 zu synchronisieren und zu steuern. Die Aufnahmesteuerungseinrichtung 5 steht auch in Datenaustauschverbindung mit einer Datenverarbeitungseinheit 6, 7, die hier durch zwei EDV-Anlagenmodule 6 und 7 dargestellt ist. Das erste Modul 6 der Datenverarbeitungseinrichtung empfängt und verarbeitet die Signale des Farbkamerasensors und erzeugt daraus aufeinanderfolgende Bilder (Video-Frames). Jedes aufeinanderfolgende Bild stellt ein spektrales Belichtungsmuster aus einer Aktivierungssequenz mit mehreren aufeinanderfolgenden, unterschiedlichen spektralen Belichtungsmustern. Die Aktivierungssequenzen werden ihrerseits durch die Aufnahmesteuerungseinrichtung zeitlich aufeinanderfolgend wiederholt. Jedem von dem Modul 6 aufgenommenen Bild des RGB-Farbkamerasensors entspricht also ein bestimmtes spektrales Belichtungsmuster aus der Aktivierungssequenz. Die aufeinanderfolgenden Bilder der spektralen Beleuchtungsmuster einer Aktivierungssequenz werden in dem Analyse-Modul 7 der Datenverarbeitungseinrichtung mit vorgegebenen Algorithmen einer multispektralen Analyse unterzogen, um daraus gewünschte Informationen und physiologische Parameter abzuleiten, wie weiter unten beschrieben wird. Dem Analyse-Modul 7 der Datenverarbeitungseinrichtung können auch weitere Funktionen wie Bildspeicherung, Bildanalyse und weitere Funktionen zugeordnet sein. Das Analyse-Modul 7 der Datenverarbeitungseinrichtung kann z.B. auf einem PC basieren. Das Datenaufnahmemodul 6 kann grundsätzlich auch in diesem PC realisiert sein, in der Regel ist das Datenaufnahmemodul 6in einem Echtzeit-Prozessorsystem implementiert.

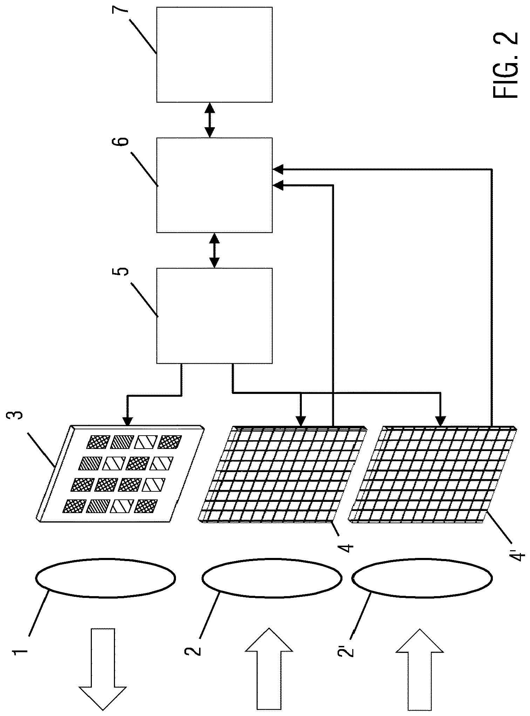

Fig. 2 zeigt ein entsprechendes Blockschaltbild, wobei die dort skizierte medizinische Bildgebungsvorrichtung stereoskopische Aufnahmen liefert. Dazu ist sie mit einem weiteren Objektiv 2' und einem weiteren RGB-Farbkamerasensor 4' versehen, wobei die beiden Objektive und die zugehörigen RGB-Farbkamerasensoren 4' so angeordnet sind, dass aus den Aufnahmen der beiden RGB-Farbkameras 4, 4' in der Datenverarbeitungseinrichtung 6, 7 stereoskopische Bildinformationen abgeleitet werden, um eine dreidimensionale Bildgebung zu erreichen. In dieser Ausführungsform bestimmt der RGB-Farbkamerasensor 4 als Master die Synchronisation, während der zweite RGB-Farbkamerasensor 4' und die synchronisierte multispektrale Lichtquelle 3 als Slave betrieben werden.

Die Lichtquelle 3 umfasst mehrere LED-Emitter im sichtbaren und im NIR-Spektralbereich. LEDs sind schnell schaltbare, variable Emitter (typisch sind Lichtpulse mit einer Länge im Bereich von 10 µs bis 10.000 µs). LEDs arbeiten ohne thermische Probleme mit hohen, aber für das Gewebe unkritischen Lichtleistungen. Vorzugsweise umfasst die Lichtquelle mehr als 10 LEDs, so dass mehr als 10 Stützstellen-Wellenlängen verteilt über den abgedeckten Spektralbereich zur Verfügung stehen. Im sichtbaren Spektralbereich sollten Stützstellen bei lokalen Absorptionsmaxima von oxygeniertem Hämoglobin vorhanden sein. Dies erlaubt eine zusätzliche Überprüfung, ob es sich bei einem vermeintlich als Pulssignal erkannten periodischen Signal tatsächlich um das Pulssignal des Kreislaufs handelt, da das echte Pulssignal mit einem entsprechenden Pulssignal für oxygeniertes Hämoglobin einhergeht.

Es ist weiter bevorzugt, dass die Lichtquelle wenigstens einen Emitter mit Emissionsmaximum im Bereich von 500 nm bis 540 nm und einen im Bereich von 570 nm bis 600 nm umfasst. Insbesondere können ein Emitter mit einer Wellenlänge von etwa 520 nm und ein Emitter mit einer Wellenlänge von 585 nm vorhanden sein. Diese Wellenlängen erlauben eine gut Diskriminierung zwischen oxygeniertem und desoxygeniertem Hämoglobin, da 520 nm ein isosbestischer Punkt des Hämoglobins ist, d.h. die Absorptionskoeffizienten von oxygeniertem und desoxygeniertem Hämoglobin sind gleich groß. Ein weiterer isosbestischer Punkt von Hämoglobin befindet sich bei 810 nm. Demgegenüber ist die Absorption von desoxygeniertem Hämoglobin bei 575 nm und 880 nm wesentlich kleiner als die von oxygeniertem Hämoglobin. Bei 600 nm und 760 nm ist wiederum die Absorption von oxygeniertem Hämoglobin kleiner, und daher können auch diese vier Wellenlängen für die Berechnung der Oxygenierung des Hämoglobins verwendet werden.

Durch den Einsatz von kurzwelligen Emittern (violett) kann die Bildgebungsvorrichtung zur Erhöhung der Kontraste zwischen organischen Strukturen wie Blutgefäßen, Strukturen mit unterschiedlichen Wassergehalten oder Konzentrationsunterschieden anderer Chromophore verwendet werden. Durch die hohen Absorptionskoeffizienten des Hämoglobins in diesem Spektralbereich bei gleichbleibenden Streukoeffizienten des Gewebes ergeben sich sehr scharfe Bilder, da das Licht wenig in das Gewebe eindringt und gestreut wird.

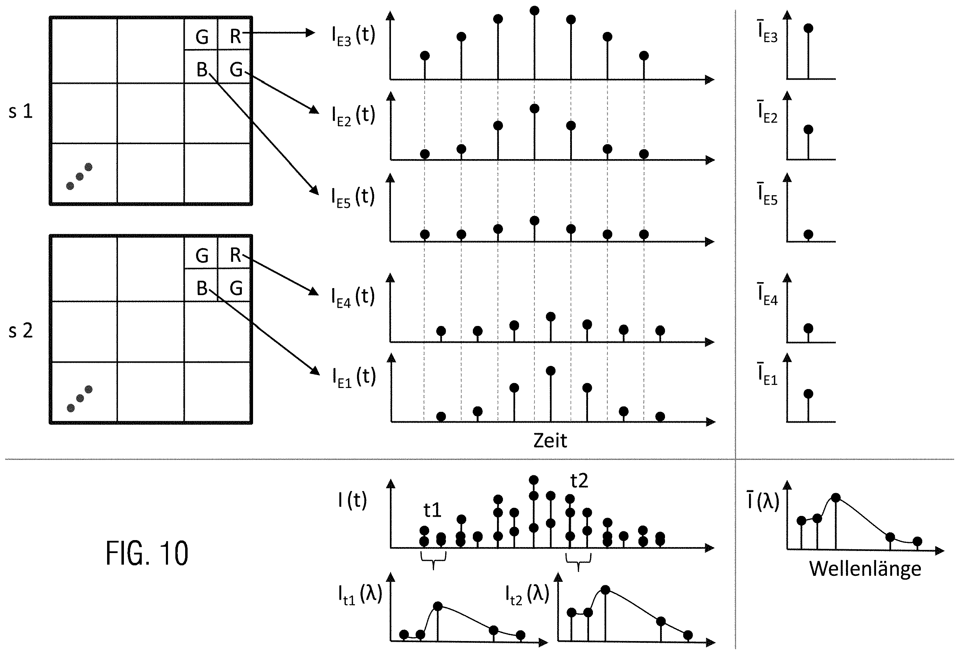

Eine bevorzugte Auswahl von Wellenlängen als spektrale Stützstellen sind in der Tabelle 1 aufgeführt und in Fig. 10 dargestellt.

**Tabelle 1: Bevorzugte Emissionswellenlängen der Lichtquelle**

| Wellenlänge in nm | Funktion |
|---|---|
| 405 | • Hämoglobinabsorption für Kontrastverstärkung |
| | • Anregung von Fluoreszenz im Rotkanal |
| 430 | • Hämoglobinabsorption für Kontrastverstärkung |
| | • Anregung von Fluoreszenzfarbstoffen und Autofluoreszenz im Gewebe |
| 455 | • Farbbild (Blaukanal) |
| 490 | • Farbbild (CRI) |
| | • Oximetriewellenlänge |
| | • Spaltung in 2 Wellenlängen durch Farbfilter des Kamerasensor(Blau Grün) |
| 520 | • Farbbild (Grünkanal) |
| | • Isosbestischer Punkt Hämoglobinabsorption |
| 540 | • Unterstützung Farbvideo und zusätzlicher Stützpunkt |
| 600 | • Farbbild (CRI) |
| | • Oximetriewellenlänge |
| | • Spaltung in 2 Wellenlängen durch Farbfilter des Kamerasensor(Grün Rot) |
| 620 | • Farbbild (Rotkanal 1) Oximetriewellenlänge |
| | • Pulsoximetrie |
| 660 | • Farbbild (Rotkanal 2) Oximetriewellenlänge |
| | • Pulsoximetrie |
| 760 | • Lokales Absorptionsmaximum von desoxygeniertem Hämoglobin |
| 810 | • Isosobestischer Punkt Hämoglobinabsorption |
| 880 | • Pulsoximetrie |
| | • Referenzwellenlänge für Hämoglobin-, Wasser- und Fettabsorption |
| 930 | • Lokales Absorptionsmaximum von Fett |
| 960 | • Lokales Absorptionsmaximum von Wasser |

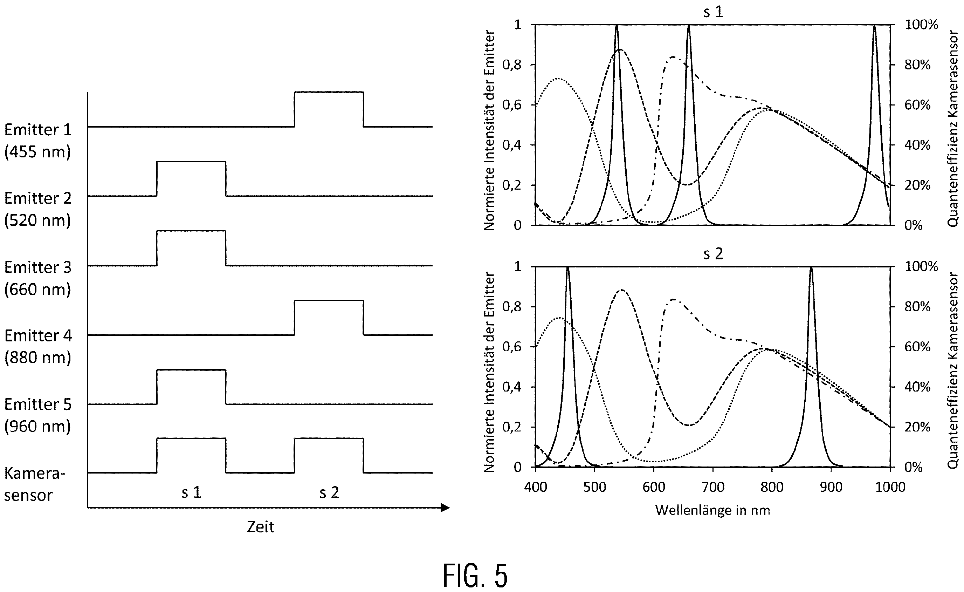

Die Aufnahmesteuereinrichtung ist z.B. durch Programmierung dazu eingerichtet in einer vorgegebenen Aktivierungssequenz eine Folge von spektralen Belichtungsmustern zu erzeugen, wobei in jedem einzelnen spektralen Belichtungsmuster ein Emitter oder mehrere Emitter mit verschiedenen Wellenlängen aktiviert werden, und diese Aktivierungssequenz zeitlich aufeinanderfolgend zu wiederholen. Jedes spektrale Belichtungsmuster der Aktivierungssequenz wird mit einem Bild des Farbkamerasensors aufgenommen. Für diese Synchronisation zwischen Lichtquelle 3 und Farbkamerasensor 4 sorgt die Aufnahmesteuereinrichtung 5. In Fig. 5 ist beispielhaft eine nicht unter die Ansprüche der Anmeldung fallende Aktivierungssequenz mit zwei aufeinanderfolgenden spektralen Belichtungsmustern S 1 und S 2 dargestellt. In dem in Fig. 5 auf der linken Seite gezeigten Graphen sind für fünf Emitter deren Aktivierung während zwei aufeinanderfolgenden spektralen Belichtungsmuster S 1 und S 2 sowie die Aufnahmeaktivität des Farbkamerasensors als Funktion der Zeit dargestellt. Bei einer maximalen Bildwiederholrate des Farbkamerasensors von 120 Hz werden hier zwei spektrale Belichtungsmuster (das erste mit gleichzeitiger Aktivierung von Emittern mit 520, 660 und 960 nm, und das zweite mit gleichzeitiger Aktivierung von zwei Emittern bei 455 und 880 nm) in einer Aktivierungssequenz nacheinander wiederholt erzeugt. Somit stehen durch effiziente Wahl der Emitter und unter Ausnutzung der drei Farbbilder des Farbkamerasensors fünf spektrale Stützstellen-Wellenlängen zur Verfügung, wobei pro Aktivierungssequenz zwei Bilder durch den Farbkamerasensor aufgenommen werden, so dass die Bildwiederholrate für die multispektrale Aufnahme mit fünf Stützstellen-Wellenlängen 60 Hz beträgt. Diese spektralen Wellenlängen-Stützstellen können beispielsweise für die gleichzeitige Berechnung eines Farbvideobildes, ortsaufgelöste SpO₂-Darstellung und ortsaufgelöste Darstellung des Gewebewasserindex verwendet werden.

Die Anzahl von spektralen Belichtungsmustern und die Auswahl der in den jeweiligen Belichtungsmustern zu aktivierenden Emitter kann während des Betriebs der Aufnahmesteuerungseinrichtung verändert werden. Dazu kann eine Eingabevorrichtung vorgesehen sein, in die der Benutzer die Anzahl von spektralen Belichtungsmustern in der Aktivierungssequenz, und dann für jedes spektrale Belichtungsmuster jeweils die zu aktivierenden Emitter eingibt. Somit kann bei Bedarf eine höhere Anzahl von spektralen Stützstellen-Wellenlängen bei niedrigerer Bildwiederholrate oder umgekehrt eine geringere Anzahl von spektralen Stützstellen-Wellenlängen bei einer höheren Bildwiederholrate aufgenommen werden.

Die in Fig. 5 illustrierte Aktivierungssequenz mit zwei aufeinanderfolgenden spektralen Belichtungsmustern kann zur Erzeugung eines Farbbildes (Farbvideo), eines physiologischen Parametervideos, das die Wasserkonzentration darstellt (Wasserabsorptionsbande 960 nm zu Referenzpunkt 880 nm) und zur Erzeugung eines Parametervideos für SpO₂ verwendet werden, wobei letzteres aus dem pulsatilen Teil des Signals bei 660 nm, 880 nm und 960 nm extrahiert wird. Während des ersten spektralen Belichtungsmusters sind Emitter 2 (520 nm), Emitter 3 (660 nm) und Emitter 5 (960 nm) angeschaltet. Die normierte Intensität der Emitter und die Farbfilterkurven des Farbkamerasensors sind in dem Graphen oben rechts dargestellt. Der größte Signalanteil im blauen Farbsignal stammt von Emitter 5. Da die Sensitivität der Farbkamerasensors oberhalb von 800 nm in den drei Farbkanälen R, G und B nahezu identisch ist, kann die Information über die Signalintensität des Emitters 5 im blauen Farbsignal genutzt werden, um die Signalintensitäten des grünen und des roten Farbsignals zu korrigieren. Um das tatsächliche Signal des Emitters 2 (520 nm) zu erhalten, wird das blaue Farbsignal von dem grünen Farbsignal subtrahiert. Für die Bestimmung des tatsächlichen Signals des Emitters 3 (660 nm) wird das gemessene blaue Farbsignal von dem roten Farbsignal subtrahiert. Somit liegen nach der Aufnahme des ersten Bildes (des ersten spektralen Belichtungsmusters S 1) bereits zwei Stützstellen im sichtbaren und eine im nahinfraroten Bereich vor. Für das zweite spektrale Belichtungsmuster S 2 werden nur die Emitter 1 (455 nm) und 4 (880 nm) angeschaltet. Das rote Farbsignal stammt nur von Emitter 4. Das Signal von Emitter 1 kann durch Subtraktion des blauen Farbsignals von dem roten Farbsignal berechnet werden. Nach Abschluss der Aktivierungssequenz mit den zwei spektralen Belichtungsmustern S 1 und S 2 stehen fünf spektrale Stützstellen-Wellenlängen zur Verfügung.

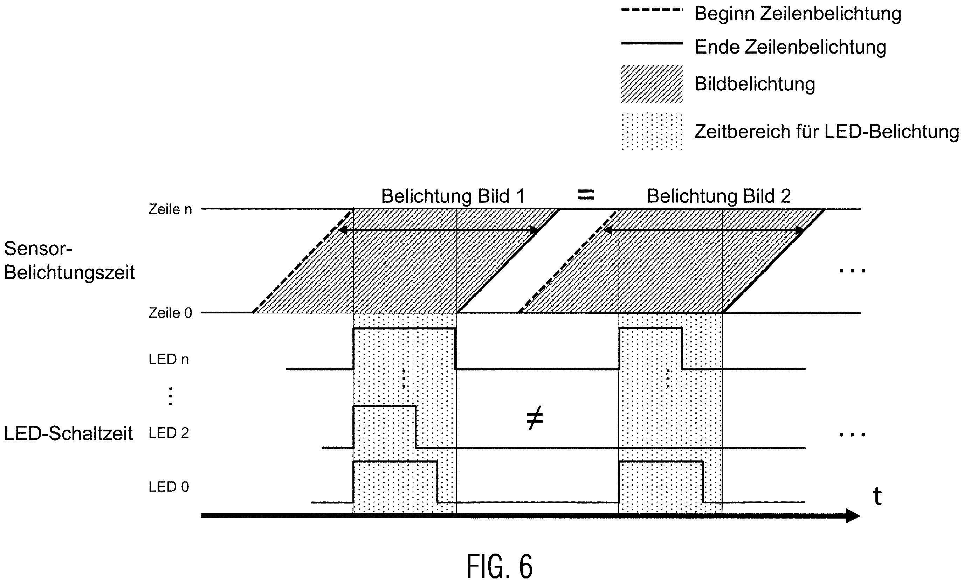

Fig. 6 zeigt die zeitliche Abfolge von Auslesen und Belichtung von CMOS-Farbkamerasensoren, die von der Bauart Rolling-Shutter sind. Diese CMOS-Farbkamerasensoren weisen keinen globalen Shutter auf, so dass kontinuierlich und zueinander zeitversetzt einzelne Zeilen des Kamerasensorchips ausgelesen werden, wodurch es bei sequenzieller Aktivierung der LED-Emitter zu Überlappungen der Belichtung innerhalb einzelner Frames kommen kann. Zur Lösung dieses Problems wird eine gewisse Totzeit zwischen aufeinanderfolgenden spektralen Belichtungsmustern der LEDs vorgesehen, die wenigstens der Auslesezeit und/oder Reset-Zeit des CMOS-Sensors entspricht. Alternativ ist denkbar, dass die Belichtungszeiten der spektralen Belichtungsmuster größer gewählt werden als die Zeitabstände zwischen einem Reset und einem Auslesen des gesamten CMOS-Sensors.

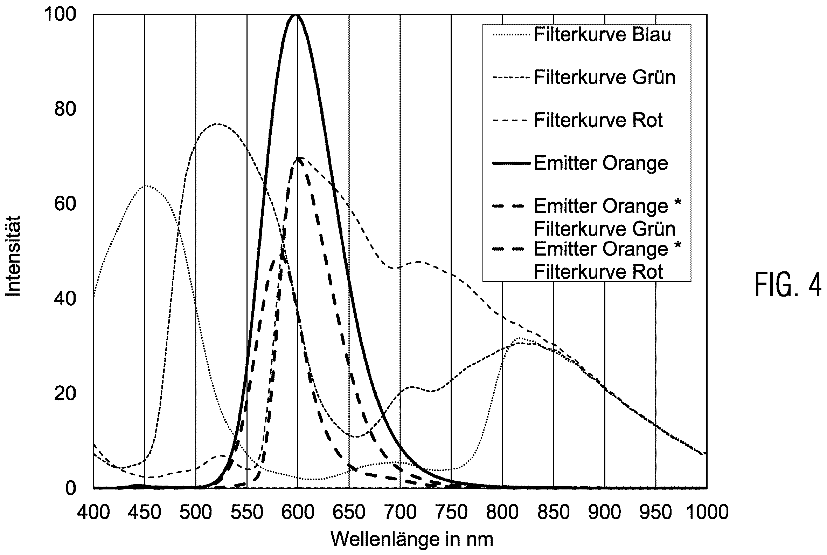

Fig. 4 illustriert die Erzeugung von zwei spektralen Stützstellen aus der Emissionskurve eines einzelnen Emitters (Emitter orange) und den Farbfilterkurven des Farbkamerasensors. Die Wellenlänge des orangen Emitters (dessen Spitzenwellenlänge liegt nahe an dem Schnittpunkt von Grün-Filterkurve und Rot-Filterkurve). Durch die gegenläufig verlaufenden Grün- und Rot-Filterkurven werden die resultierenden Signalmaxima im grünen und im roten Farbsignal um einige Nanometer entgegengesetzt zueinander verschoben. Das Signalmaximum im grünen Farbsignal ist gegenüber dem Maximum der Emissionskurve des orangenen Emitters zu kleineren Wellenlängen hin verschoben, während das Signalmaximum im roten Farbsignal gegenüber dem orangenen Emitter zu größeren Wellenlängen hin verschoben ist. Effektiv wird die Emissionskurve des Emitters also im Schnittpunktbereich der Filterkurven durch die beiden Filterkurven in zwei Wellenlängenmaxima in den beiden Farbsignalen zerlegt. Durch die Zerlegung der Emissionskurve in zwei benachbarte Signalmaxima in den resultierenden Farbsignalen kann unter Verwendung eines einzelnen Emitters die Anzahl der auswertbaren Wellenlängen (Stützstellen) für die multispektrale Analyse effektiv erhöht werden.

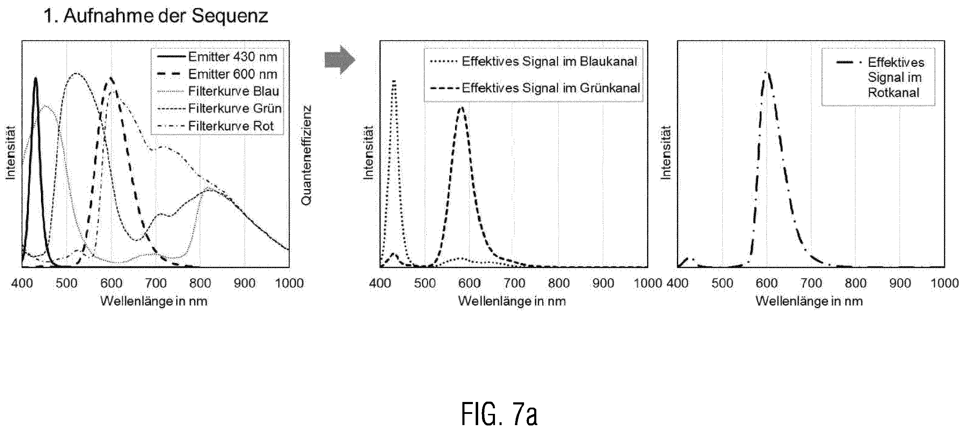

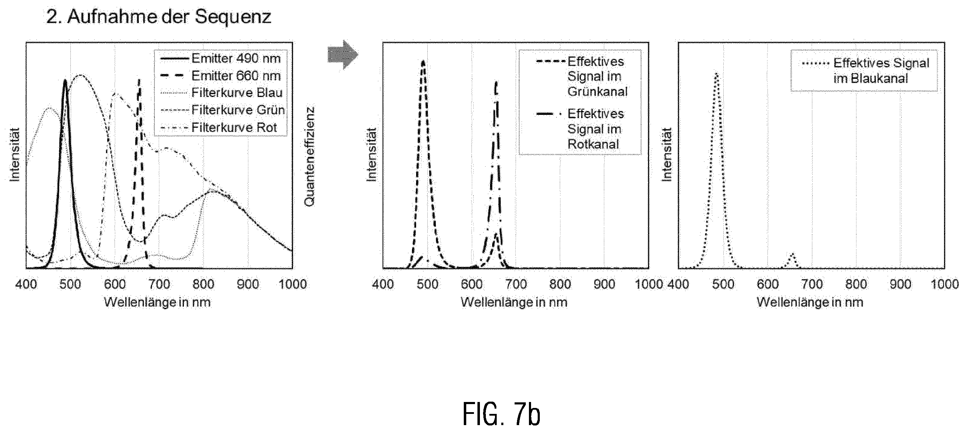

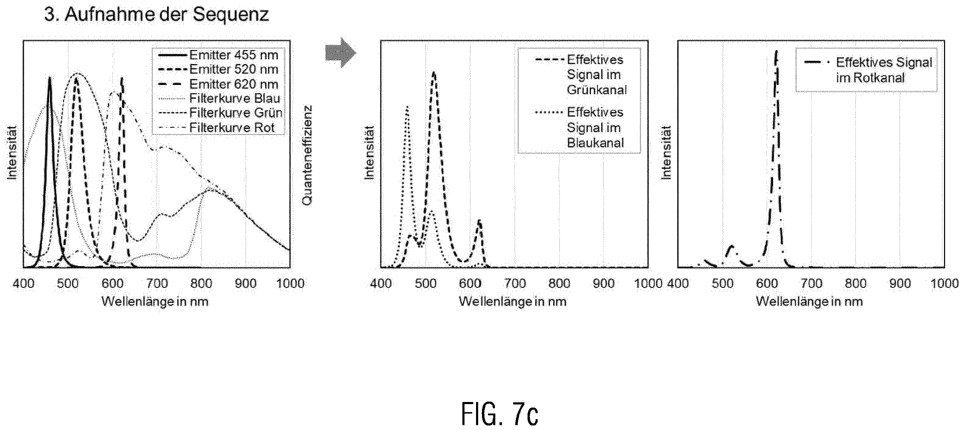

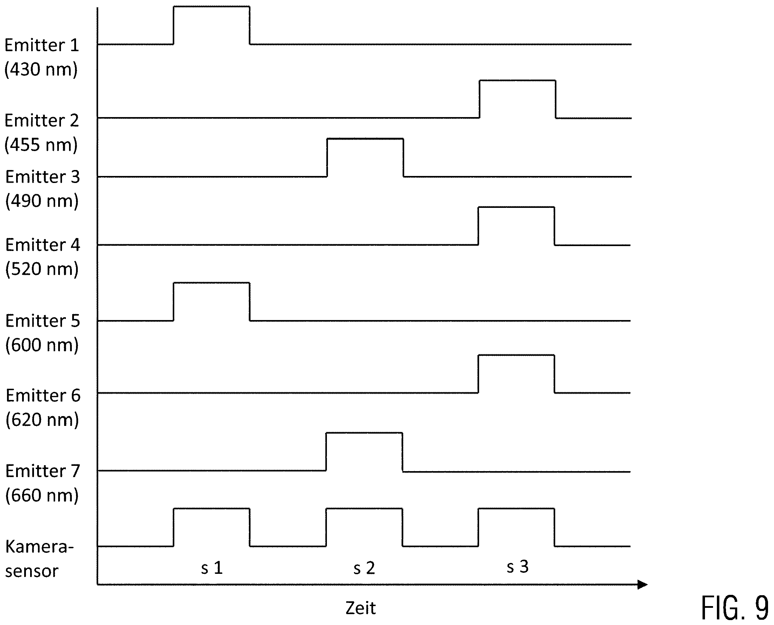

Fig. 7a - 7c illustrieren eine Aktivierungssequenz, die den eben geschilderten Effekt der Aufspaltung des Spektrum eines Emitters nahe am Schnittpunkt von Grün- und Rot-Filterkurve in gegeneinander verschobene grüne und rote Farbsignale ausnutzt. Eine derartige Aktivierungssequenz ist in einer erfindungsgemäßen Bildgebungsvorrichtung anwendbar. Die Aktivierungssequenz mit drei aufeinanderfolgenden spektralen Belichtungsmustern ist in Fig. 7a bis 7c jeweils in Form von Emissionsspektren der beteiligten Emitter und der resultierenden Farbsignale für das erste (Fig. 7a), das zweite (Fig. 7b) und das dritte spektrale Belichtungsmuster (Fig. 7c) gezeigt; Fig. 9 illustriert diese spektralen Belichtungsmuster in Form eines Graphen der Aktivierungsperioden der einzelnen Emitter und des Kamerasensors als Funktion der Zeit über die drei aufeinanderfolgenden spektralen Belichtungsmuster S 1, S 2 und S 3. Insbesondere zeigt Fig. 9 die zeitliche Abfolge der Schaltflanken während der drei spektralen Belichtungsmuster mit insgesamt sieben Emittern wie in Fig. 7a bis 7c dargestellt. Während des Zeitraums S 1 des ersten spektralen Belichtungsmusters sind Emitter 1 (430 nm) und Emitter 5 (600 nm) angeschaltet. Während des zweiten spektralen Belichtungsmusters (S 2) sind Emitter 3 (490 nm) und Emitter 7 (660 nm) eingeschaltet. Für das dritte spektrale Belichtungsmuster über den Zeitraum S3 werden die Emitter 2 (455 nm), Emitter 4 (520 nm) und Emitter 6 (620 nm) angeschaltet. Der Kamerasensor wird während der Phasen aller drei spektralen Belichtungsmuster zur Bildaufnahme geschaltet.

Die Aktivierungssequenz aus Fig. 7a - 7c und 9 verwendet sieben Emitter im sichtbaren Bereich, aus denen neun spektrale Stützstellen gewonnen werden. Aus dieser Aktivierungssequenz lassen sich zum Beispiel ein Farbvideo, ein Parametervideo zur Gewebeoxygenierung (StO₂) und ein Parametervideo der relativen Gewebehämoglobinkonzentration erzeugen.

Fig. 7a bis c zeigen jeweils im linken Graphen die Emissionsspektren der verwendeten Emitter in den drei aufeinanderfolgenden spektralen Belichtungsmustern zusammen mit den Filterkurven des Farbkamerasensors. Der mittlere und der rechte Graph in Fig. 7a bis 7c zeigen die effektiven Farbsignale für die drei Farben. Das erste spektrale Belichtungsmuster der Aktivierungssequenz schaltet zwei Emitter bei 430 nm du 600 nm ein, wobei das Emissionsspektrum des letzteren Emitters nahe am Schnittpunkt der Grün- und Rot-Filterkurven liegt und dessen Emissionsspektrum daher in den resultierenden grünen und roten Farbsignalen aufgespalten wird, wie zuvor im Zusammenhang mit Fig. 4 beschrieben, so dass das erste spektrale Belichtungsmuster drei spektrale Stützstellen liefert.

Das zweite spektrale Belichtungsmuster verwendet zwei Emitter bei 490 nm und 660 nm, wobei die aus dem ersten Emitter bei 490 nm resultierenden Signale durch die Blau- und Grün-Filterkurven in zwei benachbarte Signalmaxima im blauen und grünen Farbsignal zerlegt wird, so dass auch das zweite spektrale Belichtungsmuster drei Stützstellen-Wellenlängen liefert.

Das dritte spektrale Belichtungsmuster der Aktivierungssequenz schaltet drei Emitter bei 455 nm, 520 nm und 620 nm ein, so dass auch das dritte spektrale Belichtungsmuster drei Stützstellen-Wellenlängen liefert. Somit ergibt die im Zusammenhang mit den Fig. 7 und 9 beschriebene Aktivierungssequenz insgesamt neun Stützstellen-Wellenlängen. Bei einer Bildwiederholrate von 120 Hz ergeben sich daher pro Sekunde vierzig vollständige Datensätze mit jeweils neun spektralen Stützstellen für die multispektrale Analyse.

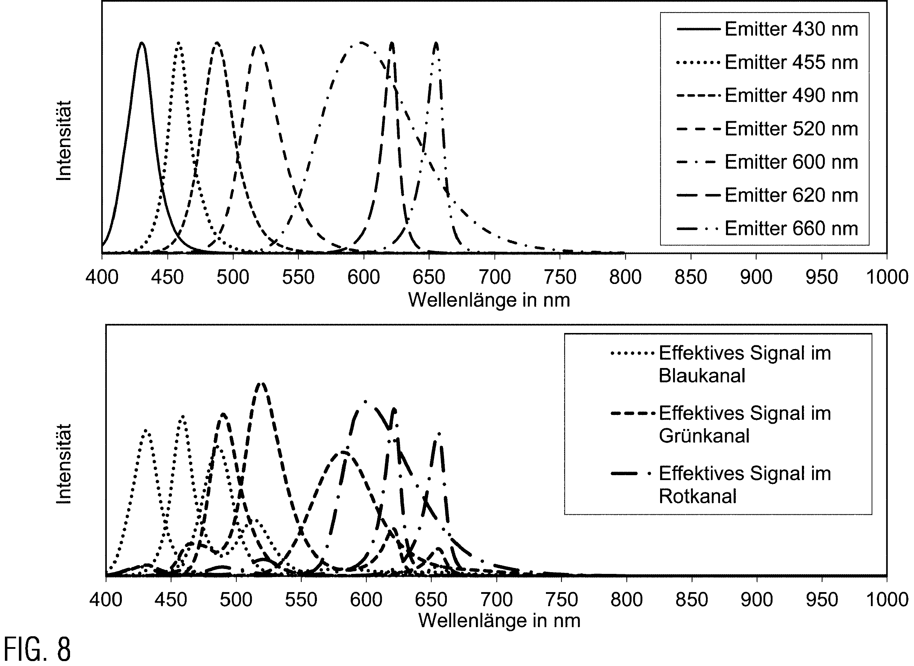

Fig. 8 zeigt in dem oberen Graphen die normierten Intensitäten der verwendeten Emitter und in dem unteren Graphen die resultierenden effektiven drei Farbsignale der oben im Zusammenhang mit den Fig. 7a bis 7c und 9 beschriebenen Aktivierungssequenz. Nach Abschluss der Aktivierungssequenz aus jeweils drei spektralen Belichtungsmustern liegen neun spektrale Stützstellen im sichtbaren Bereich von 400 bis 700 nm vor.

Fig. 10 illustriert die Aufnahme der Signale und deren Zusammenfassung für die Aktivierungssequenz aus Fig. 5, die in der Darstellung der Graphen in der mittleren Spalte von Fig. 10 siebenmal hintereinander wiederholt gezeigt ist. Die in der mittleren Spalte gezeigten oberen fünf Graphen I_{E1}(t) ... I_{E5} (t) zeigen folgende Farbsignale über sieben Aktivierungssequenzen mit je zwei spektralen Belichtungsmustern S 1 und S 2: der Graph I_{E3}(t) zeigt das auf die Emission des Emitters 3 zurückgehende Signal im roten Farbsignal (der ansteigende und wieder abfallende Verlauf über die sieben Aktivierungssequenzen geht auf die Pulsation des Hämoglobins zurück, das bei dieser Wellenlänge gut sichtbar ist); die zweite Signalfolge I_{E2}(t) zeigt das auf die Aktivierung des Emitters 2 (520 nm) zurückgehende Signal im grünen Farbsignal; das dritte Signal I_{E5}(t) zeigt das auf die Aktivierung des Emitters 5 (960 nm) zurückgehende Signal im blauen Farbsignal. Diese ersten drei Signale werden jeweils simultan im ersten spektralen Belichtungsmuster S 1 der aufeinanderfolgenden Aktivierungssequenzen aufgenommen. Das vierte Signal I_{E4}(t) geht auf die Aktivierung des Emitters 4 (880 nm) zurück und wird im roten Farbsignal aufgenommen. Jeweils simultan dazu wird in dem zweiten spektralen Belichtungsmuster S 2 das auf die Aktivierung des Emitters 1 (455 nm) zurückgehende Signal als blaues Farbsignal aufgenommen. Da das zweite spektrale Belichtungsmuster zeitlich dem ersten spektralen Belichtungsmuster jeder Aktivierungssequenz zeitversetzt nachfolgt sind die Signale I_{E4}(t) und I_{E1}(t) gegenüber den Signalen des ersten spektralen Belichtungsmusters in den drei oberen Graphen zeitversetzt.

In der rechten Spalte sind die zeitlich gemittelten Signale dargestellt (d.h. die über die sieben Aktivierungsphasen, die in den Graphen in der mittleren Spalte dargestellt sind, zeitlich gemittelten Signale bei den jeweiligen Wellenlängen). Aus diesen mittleren Signalen bei den einzelnen Wellenlängen kann der Graph I̅(λ) mit allen fünf spektralen Stützstellen zusammengesetzt werden (rechts unten in Fig. 10), der als Gewebespektrum ausgewertet werden kann. Dieses Gewebespektrum kann für die Berechnung von nicht-pulsatilen physiologischen Gewebeparametern verwendet werden, bei denen eine zeitliche Mittelung zu Erhöhung der Signalqualität notwendig ist.

Der Graph I(t), der als zweiter von unten in der mittleren Spalte von Fig. 10 dargestellt ist, zeigt die gemessenen Intensitäten aller Farbsignale und die jeweils aufeinanderfolgenden ersten und zweiten spektralen Belichtungsmuster über die sieben aufeinanderfolgenden Aktivierungssequenzen aller drei Farbsignale. Aus der Differenz der Intensitäten von zwei zeitlich auseinander liegenden Aktivierungssequenzen können weitere Informationen gewonnen werden. In den beiden untersten Graphen der mittleren Spalte von Fig. 10 sind die Intensitäten Iₜ₁(λ) als Funktion der Wellenlänge und die Intensität Iₜ₂(λ) als Funktion der Wellenlänge in zwei zeitlich auseinander liegenden Aktivierungssequenzen dargestellt. Mit anderen Worten zeigt Iₜ₁(λ) die fünf spektralen Stützstellen, die in der ersten Aktivierungssequenz aufgenommen worden sind, und der Graph Iₜ₂(λ) die fünf spektralen Stützstellen, die in der fünften Aktivierungssequenz aufgenommen worden sind. Aus der Differenz der Stützstellen in den beiden Graphen Iₜ₁(λ) - Iₜ₂(λ) kann der pulsatile Anteil des Signals (zeitlich veränderlicher Anteil) abgeleitet werden. Dieser pulsatile Anteil kann für die Erzeugung des physiologischen Parameterbildes für SpO2, welches auf dem pulsatilen Teil des Signals bei 660 nm, 880 nm und 960 nm extrahiert wird, verwendet werden.

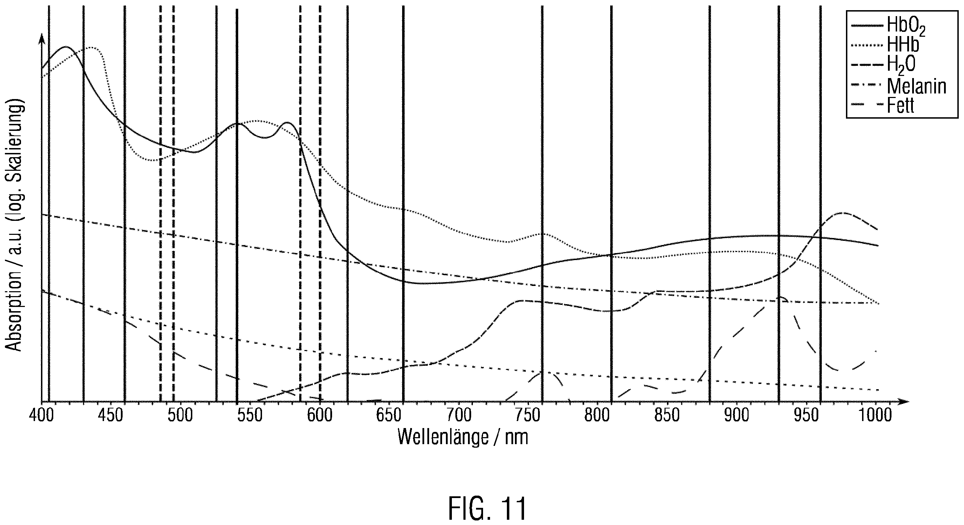

Fig. 11 zeigt die Absorptionskurven der Hauptabsorber im Gewebe im sichtbaren und nahinfraroten Bereich. Zusätzlich sind die bevorzugten Emissionswellenlängen der Emitter der Lichtquellen und die daraus gegebenenfalls in Kombination mit dem Filterkurven des Farbkamerasensors resultierenden effektiven Stützstellen-Wellenlängen dargestellt.

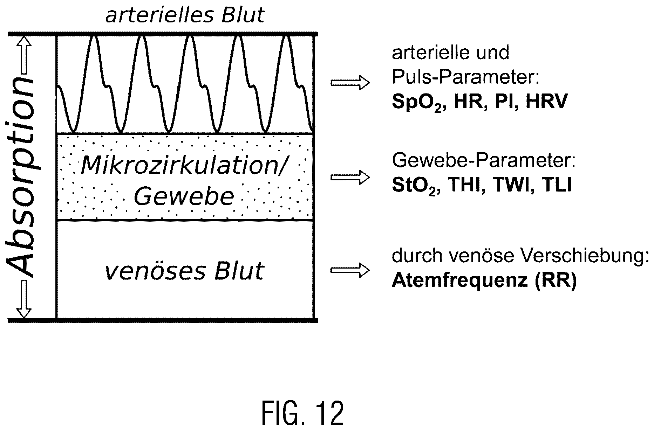

In Fig. 12 sind die pulsatilen und nicht-pulsatilen Signalanteile der Gewebeabsorption dargestellt. Der pulsatile Anteil wird durch das arterielle Blut verursacht und kann, nach der Trennung vom nicht-pulsatilen Signalanteil, dazu verwendet werden, um die Sauerstoffsättigung des arteriellen Blutes (SpO2), die Herzfrequenz (HR), den Pulsationsindex (PI) und die Herzratenvariabilität (HRV) zu bestimmen. Der nicht-pulsatile Signalanteil enthält unter anderem Informationen über die Sauerstoffsättigung der Mikrozirkulation im Gewebe (StO2), den Gewebehämoglobingehalt (THI), den Gewebewassergehalt (TWI) und den Gewebefettgehalt (TLI). Ein weiterer Signalanteil wird durch die Verschiebung des venösen Bluts verursacht und kann unter anderem zur Bestimmung der Atemfrequenz (RR) verwendet werden.

## Patentansprüche

1. Medizinische Bildgebungsvorrichtung zur ortsaufgelösten Aufnahme multispektraler Videodaten eines Untersuchungsgebiets eines Patienten, mit
einer Lichtquelle (3) zur Beleuchtung des Untersuchungsgebiets, die mehrere optische Emitter mit unterschiedlichen Wellenlängen verteilt über den sichtbaren und NIR-Spektralbereich aufweist,
einem RGB-Kamerasensor (4) zur Aufnahme des Untersuchungsgebiets, der mit Filtern mit Rot-, Grün- und Blau-Filterkurven rote, grüne und blaue Farbsignale erzeugt,
einer Aufnahmesteuerungseinrichtung (5), die zur synchronisierten Steuerung von Lichtquelle (3) und RGB-Kamerasensor (4) eingerichtet ist, um einen oder mehrere Emitter, die zusammen ein spektrales Belichtungsmuster mit einer oder mehreren Wellenlängen erzeugen, in einer vorgegebenen Aktivierungssequenz mehrerer aufeinanderfolgender spektraler Belichtungsmuster, die nach Reflexion im Untersuchungsgebiet jeweils als Bild durch den RGB-Kamerasensor aufgenommen werden, wiederholt zu aktivieren,
einer mit der Aufnahmesteuerungseinrichtung (5) und dem RGB-Kamerasensor (4) verbundenen Datenverarbeitungseinrichtung (6, 7), die zur Aufnahme der Farbsignale des RGB-Kamerasensors und zu deren Auswertung zur ortsaufgelösten multispektralen Analyse zur Ableitung eines physiologischen Parameters und zu dessen ortsaufgelöster Darstellung als Video des Untersuchungsgebiets eingerichtet ist,
**dadurch gekennzeichnet, dass**
die Lichtquelle (3) einen Emitter aufweist, dessen Wellenlänge im Bereich von ±50% seiner Halbwertsbreite um den Schnittpunkt der Blau- und Grün-Filterkurve oder der Grün- und Rot-Filterkurve liegt, und
die Aufnahmesteuerungs- (5) und die Datenverarbeitungseinrichtung (6, 7) dazu eingerichtet sind, in einem Belichtungsmuster mit Aktivierung des Emitters am Schnittpunkt die betroffen beiden der roten und grünen oder der grünen und blauen Farbsignale separat zu erfassen und diese in der multispektralen Analyse mit durch die beiden betroffenen Filterkurven gegeneinander verschobenen, voneinander verschiedenen Wellenlängen als zwei Stützstellen-Wellenlängen auszuwerten.

2. Medizinische Bildgebungsvorrichtung nach Anspruch 1, **dadurch gekennzeichnet, dass**
die Lichtquelle (3) einen ersten Emitter aufweist, dessen Wellenlänge im Bereich von ±50% seiner Halbwertsbreite um den Schnittpunkt der Blau- und Grün-Filterkurve liegt, und einen zweiten Emitter aufweist, dessen Wellenlänge im Bereich von ±50% seiner Halbwertsbreite um den Schnittpunkt der Grün- und Rot-Filterkurve liegt, und
die Aufnahmesteuerungs- (5) und die Datenverarbeitungseinrichtung (6, 7) dazu eingerichtet sind, den ersten und den zweiten Emitter in verschiedenen Belichtungsmustern der Aktivierungssequenz zu aktivieren und bei Aktivierung des ersten Emitters die grünen und blauen Farbsignale separat zu erfassen und diese in der multispektralen Analyse mit durch die Blau- und Grün-Filterkurve gegeneinander verschobenen, voneinander verschiedenen Wellenlängen als zwei Stützstellen-Wellenlängen auszuwerten und bei Aktivierung des zweiten Emitters die grünen und roten Farbsignale separat zu erfassen und diese in der multispektralen Analyse mit durch die Grün- und Rot-Filterkurve gegeneinander verschobenen, voneinander verschiedenen Wellenlängen als zwei Stützstellen-Wellenlängen auszuwerten.

3. Medizinische Bildgebungsvorrichtung nach Anspruch 1, **dadurch gekennzeichnet, dass**
die Lichtquelle (3) einen ersten Emitter aufweist, dessen Wellenlänge um weniger als ±50% seiner Halbwertsbreite kleiner als die Wellenlänge am Schnittpunkt der Blau- und Grün-Filterkurve ist, und einen zweiten Emitter aufweist, dessen Wellenlänge um weniger ±50% seiner Halbwertsbreite größer als die Wellenlänge am Schnittpunkt der Blau- und Grün-Filterkurve ist, und
die Aufnahmesteuerungs- (5) und die Datenverarbeitungseinrichtung (6, 7) dazu eingerichtet sind, den ersten und den zweiten Emitter in verschiedenen Belichtungsmustern der Aktivierungssequenz zu aktivieren und sowohl bei Aktivierung des ersten Emitters als auch bei Aktivierung des zweiten Emitters jeweils die grünen und blauen Farbsignale separat zu erfassen und diese in der multispektralen Analyse jeweils mit durch die Blau- und Grün-Filterkurve gegeneinander verschobenen, voneinander verschiedenen Wellenlängen als insgesamt vier Stützstellen-Wellenlängen auszuwerten.

4. Medizinische Bildgebungsvorrichtung nach einem der vorhergehenden Ansprüche, **dadurch gekennzeichnet, dass** die Aufnahmesteuerungs- (5) und die Datenverarbeitungseinrichtung (6, 7) dazu eingerichtet sind, bei Steuerung der Lichtquelle (3) mit einem Belichtungsmuster mit einem oder mehreren Emittern mit Wellenlängen im sichtbaren Bereich gleichzeitig einen Emitter im NIR-Spektralbereich zu aktivieren und dasjenige von den blauen, grünen und roten Farbsignalen, auf das die Belichtung im sichtbaren Bereich den geringsten Einfluss hat, von den beiden anderen Farbsignalen zu subtrahieren, um den Effekt der Belichtung im NIR-Spektralbereich auf die beiden anderen Farbsignale zu kompensieren.

5. Medizinische Bildgebungsvorrichtung nach Anspruch 4, **dadurch gekennzeichnet, dass** die Aufnahmesteuerungs- (5) und Datenverarbeitungseinrichtung (6, 7) dazu eingerichtet sind, bei Steuerung der Lichtquelle (3) zur Erzeugung eines Belichtungsmusters mit Wellenlängen oberhalb von 500 nm durch einen ersten Emitter mit einer Wellenlänge im Bereich von 500 bis 600 nm und durch einen zweiten Emitter mit einer Wellenlänge im Bereich von 600 bis 700 nm und gleichzeitig durch einen Emitter mit einer Wellenlänge im NIR-Spektralbereich das blaue Farbsignal von dem grünen und dem roten Farbsignal zu subtrahieren, um den Effekt der Belichtung im NIR-Spektralbereich auf das grüne und das rote Farbsignal zu kompensieren.

6. Medizinische Bildgebungsvorrichtung nach einem der vorhergehenden Ansprüche, **dadurch gekennzeichnet, dass** die Aufnahmesteuereinrichtung (5) dazu eingerichtet ist, zwischen einem Weißlicht-Betriebsmodus und einem multispektralen Betriebsmodus hin und her schaltbar zu sein, wobei in dem Weißlicht-Betriebsmodus eine Mehrzahl von Emittern mit Wellenlängen verteilt über den sichtbaren Spektralbereich gleichzeitig kontinuierlich aktiviert wird, um eine Beleuchtung mit einem näherungsweise Weißlicht entsprechenden Spektrum zu bewirken, und dabei die roten, grünen und blauen Farbsignale des RGB-Kamerasensors (4) aufzunehmen und diese als Farbvideo zur Darstellung zu bringen, und wobei die Aufnahmesteuerungseinrichtung (5) im multispektralen Betriebsmodus dazu eingerichtet ist, in der Aktivierungssequenz Belichtungsmuster mit aktivierten Emittern zu selektieren, die bei Summation ein angenähertes Weißlichtspektrum ergeben, und die zugehörigen summierten roten, grünen und blauen Farbsignale als Farbvideo mit gegenüber dem Farbvideo im Weißlicht-Betriebsmodus reduzierter Bildrate parallel neben dem ortsaufgelösten Video des durch die multispektrale Analyse abgeleiteten physiologischen Parameters darzustellen.

7. Medizinische Bildgebungsvorrichtung nach einem der vorhergehenden Ansprüche, **dadurch gekennzeichnet, dass** die Aufnahmesteuerungs- (5) und die Datenverarbeitungseinrichtung (6, 7) dazu eingerichtet sind, die im Zuge der multispektralen Analyse für mehrere Wellenlängen bestimmten Intensitäten *I_{λi}*(*t*) jeweils über die Belichtungsmuster mehrerer aufeinanderfolgender Aktivierungssequenzen zu einem zeitlichen Mittel *̅I̅_̅{̅λ̅i̅}̅*̅(̅*̅t̅*̅)̅ zusammenzufassen, daraus den Graphen eines Gewebespektrums mit den Stützstellen der mehreren Wellenlängen *λᵢ* zu bilden und daraus als physiologischen Parameter einen nicht-pulsatilen, gewebespezifischen Parameter abzuleiten.

8. Medizinische Bildgebungsvorrichtung nach Anspruch 7, **dadurch gekennzeichnet, dass** die Aufnahmesteuerungs- (5) und Datenverarbeitungseinrichtung (6, 7) dazu eingerichtet sind, als nicht-pulsatilen Parameter die Sauerstoffsättigung der Mikrozirkulation im Gewebe, den Gewebehämoglobingehalt, den Gewebewassergehalt oder den Gewebefettgehalt zu bestimmen.

9. Medizinische Bildgebungsvorrichtung nach einem der vorhergehenden Ansprüche, **dadurch gekennzeichnet, dass** die Aufnahmesteuerungs- (5) und die Datenverarbeitungseinrichtung (6, 7) dazu eingerichtet sind, die im Zuge der multispektralen Analyse für mehrere Wellenlängen *λᵢ* bestimmten spektralen Intensitäten für eine erste Aktivierungssequenz als *I*_{*t*1}(*λi*) aufeinanderfolgen zu erfassen und für eine spätere, zweite Aktivierungssequenz als *I*_{*t*2}(*λᵢ*) zu erfassen und den Graphen der an den Stützstellen-Wellenlängen *λᵢ* gebildeten Differenz der Intensitäten *I*_{*t*1}(*λᵢ*) und *I*_{*t*2}(*λᵢ*) als Funktion von *λᵢ* auszuwerten, um einen pulsatilen Parameter als physiologischen Parameter zu bestimmen.

10. Medizinische Bildgebungsvorrichtung nach Anspruch 9, **dadurch gekennzeichnet, dass** die Aufnahmesteuerungs- (5) und Datenverarbeitungseinrichtung (6, 7) dazu eingerichtet sind, als pulsatilen Parameter die Sauerstoffsättigung des arteriellen Blutes, die Herzfrequenz, den Pulsationsindex oder die Herzfrequenzvariabilität zu bestimmen.

11. Medizinische Bildgebungsvorrichtung nach einem der vorhergehenden Ansprüche, **dadurch gekennzeichnet, dass** zwei RGB-Kamerasensoren (4, 4') so zueinander angeordnet sind, um stereoskopische Aufnahmen zu ermöglichen, und dass die Datenverarbeitungseinrichtung (6, 7) dazu eingerichtet ist, die Signale der beiden RGB-Kamerasensoren so auszuwerten, dass ein stereoskopisches Farbvideo zur Darstellung gebracht wird und eine stereoskopische Darstellung des durch die multispektrale Analyse ortsaufgelöst abgeleiteten physiologischen Parameters zur Anzeige gebracht wird.

12. Medizinische Bildgebungsvorrichtung nach einem der vorhergehenden Ansprüche, **dadurch gekennzeichnet, dass** eine Anregungslichtquelle zur Beleuchtung des Untersuchungsgebiets mit Anregungslicht im blauen oder im ultravioletten Spektralbereich vorhanden ist, wobei die Anregungslichtquelle so ausgestaltet ist, dass ihr Spektrum des Anregungslichts von einer der Rot-, Grün- und Blau-Filterkurven blockiert wird, so dass demgegenüber längerwelliges Fluoreszenzlicht von dem RGB-Kamerasensor (4) ungestört durch das Anregungslicht erfassbar ist.

13. Medizinische Bildgebungsvorrichtung nach einem der vorhergehenden Ansprüche, **dadurch gekennzeichnet, dass** die Bildgebungsvorrichtung ein Endoskop aufweist, das an seinem distalen Ende die Lichtquelle (3) und den RGB-Kamerasensor (4) trägt, die über durch das Endoskop hindurch geführte und aus seinem proximalen Ende austretende Leitungen mit der Aufnahmesteuerungseinrichtung (5) und der Datenverarbeitungseinrichtung (6, 7) in Datenaustauschverbindung stehen.

14. Verfahren zur Aufnahme multispektraler Videodaten eines Untersuchungsgebiets eines Patienten, zur multispektralen Analyse zur ortsaufgelösten Ableitung eines physiologischen Parameters und zu dessen ortsaufgelöster Darstellung als Video des Untersuchungsgebietes, wobei bei dem Verfahren
das Untersuchungsgebiet mit einer Lichtquelle (3), die mehrere optische Emitter mit unterschiedlichen Wellenlängen verteilt über den sichtbaren und NIR-Spektralbereich aufweist, beleuchtet wird,
das Untersuchungsgebiet mit einem RGB-Kamerasensor (4), der mit Filtern mit Rot-, Grün- und Blau-Filterkurven rote, grüne und blaue Farbsignale erzeugt, aufgenommen wird,
die Lichtquelle (3) und der RGB-Kamerasensor (4) durch eine Aufnahmesteuerungseinrichtung synchronisiert gesteuert betrieben werden, um einen oder mehrere Emitter der Lichtquelle jeweils zur Erzeugung eines spektralen Belichtungsmusters mit einer oder mehreren Wellenlängen zu aktivieren, und in einer vorgegebenen Aktivierungssequenz mehrere aufeinanderfolgende vorgegebene Belichtungsmuster zu erzeugen, deren Reflexion im Untersuchungsgebiet jeweils als ein Bild per Belichtungsmuster von dem RGB-Kamerasensor (4) aufgenommen wird, und
die Farbsignale des RGB-Kamerasensors (4) in einer Datenverarbeitungseinrichtung (6, 7), die mit der Aufnahmesteuerungseinrichtung (5) und dem RGB-Kamerasensor (4) verbunden und dazu eingerichtet ist, einer ortsaufgelösten multispektralen Analyse unterzogen werden, um einen physiologischen Parameter abzuleiten, und Videodaten zur ortsaufgelöster Darstellung des Parameters als Video des Untersuchungsgebiets zu erzeugen,
**dadurch gekennzeichnet, dass**
ein Emitter der Lichtquelle (3), dessen Wellenlänge im Bereich von ±50% seiner Halbwertsbreite um den Schnittpunkt der Blau- und Grün-Filterkurve oder der Grün- und Rot-Filterkurve liegt, in einem Belichtungsmuster aktiviert wird und die betroffenen beiden Farbsignale, die den beiden Filterkurven des Schnittpunkts, in dessen Bereich der Emitter liegt, zugeordnet sind, in einem solchen Belichtungsmuster durch die Datenverarbeitungseinrichtung (6, 7) in der multispektraler in Analyse mit durch die beiden betroffenen Filterkurven gegeneinander verschobenen, voneinander verschiedenen Wellenlängen als zwei Stützstellen-Wellenlängen ausgewertet werden.
